**Europäisches Patentamt**

**European Patent Office**

**Office européen des brevets**

(19)

(11) Veröffentlichungsnummer: **0 252 484 B1**

(12) **EUROPÄISCHE PATENTSCHRIFT**

(45) Veröffentlichungstag der Patentschrift: 16.10.91

(51) Int. Cl.⁵: **A61L 2/24**, A61L 2/26

(21) Anmeldenummer: 87109753.1

(22) Anmeldetag: 07.07.87

(54) **Medizinischer Sterilisator.**

(30) Priorität: 07.07.86 DE 3622789

(43) Veröffentlichungstag der Anmeldung:
13.01.88 Patentblatt 88/02

(45) Bekanntmachung des Hinweises auf die
Patenterteilung:
16.10.91 Patentblatt 91/42

(84) Benannte Vertragsstaaten:
AT CH FR GB IT LI

(56) Entgegenhaltungen:
DE-A- 3 447 315
DE-A- 3 447 317
US-A- 3 410 650

(73) Patentinhaber: Scharmer, Klaus, Dr.
Industriestrasse
W-5173 Aldenhoven(DE)

(72) Erfinder: Scharmer, Klaus, Dr.
Industriestrasse
W-5173 Aldenhoven(DE)

(74) Vertreter: von Creytz, Dietrich, Dipl.-Phys.
Tannenweg 25
W-5144 Wegberg(DE)

## Beschreibung

Die Erfindung betrifft einen mit Solarenergie betriebenen, medizinischen Sterilisator mit einem als Dampfdrucktopf mit Druckregelventil ausgebildeten Sterilisator.

In Gebieten mit schwacher oder gar keiner Infrastruktur, in denen also insbesondere auch Elektrizität nicht zur Verfügung steht, können medizinische Geräte und dergleichen in einem Dampfdrucktopf entkeimt werden. Der Wasser und die jeweils zu entkeimenden Teile enthaltende Dampfdrucktopf kann auf einem mit am Ort vorhandenen Heizmitteln oder mit Gas betriebenen Herd bzw. Brenner erhitzt werden. Das Beschaffen bzw. Herantransportieren des herkömmlichen Heizmaterials ist mühsam und aufwendig und erzeugt bei der Anwendung erhebliche Abwärme, die zu der ohnehin meist hohen Umgebungstemperatur noch zusätzlich unangenehm wirkt.

In den relativ heißen Zonen der Erde könnte die zum Betrieb eines medizinischen Sterilisators erforderliche Energie mit Hilfe einer Solaranlage, wie sie beispielsweise in der DE OS 28 16 945 oder in der internationalen Veröffentlichung (PCT) WO 83/02149 beschrieben wird, gewonnen werden. Um in einem solchen Falle einen nennenswerten Gesamtwirkungsgrad unter Einbezug des Wärmetauschers zwischen Primärkreislauf der Solaranlage und Dampfdrucktopf zu erhalten, müßte der letztere in spezieller Weise den Erfordernissen eines möglichst verlustfreien Wärmetausches angepaßt werden.

Von der Weltgesundheitsorganisation (WHO) sind für eine umfangreiche Impfung in den heißen Entwicklungsländern verbindliche Dampfdrucktöpfe zum Sterilisieren medizinischer Geräte und dergleichen entwickelt und anerkannt worden. Diese Dampfdrucktöpfe lassen sich nicht ohne weiteres den Forderungen eines einen annehmbaren Wirkungsgrad aufweisenden Wärmetausches mit dem Primärkreislauf einer Solaranlage anpassen. Insbesondere bereitet es erhebliche Schwierigkeiten und einen entsprechend hohen Aufwand, die jeweiligen Heizmittel so dem genormten Dampfdrucktopf anzupassen, daß der Topf auf einfache Weise an der Heizstelle auszutauschen ist.

Mit Solarenergie betriebene Sterilisatoren eingangs genannter Art werden in den DE-A-34 47 315 und 34 47 317 beschrieben. In der ersteren Druckschrift wird ein solcher Sterilisator zum automatischen Betrieb durch eine mit regenerativer Energie arbeitende Heizeinrichtung angegeben, der einer destilliertes Wasser erzeugenden Einrichtung zugeordnet ist. Diese Einrichtung arbeitet wahlweise mittels Abfallwärme aus dem Sterilisator oder unmittelbar über die Heizeinrichtung. Hauptaufgabe der bekannten Anlage ist es also, die Energie optimal zu nutzen.

In der anderen Druckschrift, nämlich in der DE-A-34 47 317, wird ein mit Solarenergie arbeitender Sterilisator beschrieben, der eine Einrichtung zum Überwachen bzw. Steuern des Sterilisationsprozesses enthält. Hierdurch soll es möglich sein, unabhängig vom schwankenden Energieangebot für die zugehörige Heizeinrichtung, bei konstantem Druck in der Anlage über eine Regelung und einen Wärmetauscher aus der zugehörigen Sterilisationskammer austretenden Dampf zu kondensieren und das Kondensat in einem Meßbecher als Maß für den Sterilisationsprozeß zu messen.

Der Erfindung liegt die Aufgabe zugrunde, einen medizinischen Sterilisator zu schaffen, der gefahrlos unter Zuhilfenahme einer Solaranlage zu betreiben ist und bei dem der jeweils ausreichend lange und hoch erhitzte, als Sterilisationsbehälter ausgebildete Dampfdrucktopf mit darin verbleibenden, entkeimten medizinischen Geräten abgekoppelt und durch den nächsten Dampfdrucktopf mit weiteren darin eingesetzten medizinischen Geraten zu ersetzen ist. Die erfindungsgemäße Lösung besteht bei dem eingangs genannten Sterilisator mit einem als Dampfdrucktopf mit Druckregelventil ausgebildeten Sterilisationsbehälter darin, daß der Dampfdrucktopf über einen automatischen Verschluß beider Leitungsstücke aufweisende Schlauchkupplungen in den primären Dampfkreislauf einer Solaranlage bei Steuerung des Gesamtsystems über das Druckregelventil des Dampfdrucktopfes unmittelbar eingeschaltet ist.

Durch die Erfindung wird erreicht, daß der in der Solaranlage unmittelbar, vorzugsweise aus Wasser, erzeugte Dampf direkt zum Sterilisieren im Dampfdrucktopf heranzuziehen ist. Wegen der direkten Einspeisung des extern- nämlich in der Solaranlage - erzeugten Dampfes in den Dampfdrucktopf werden in diesem solarbetriebenen und unmittelbar zum Sterilisieren eingesetzten Dampfkreislauf Wärmetauscher nicht benötigt.

Als weiterer Vorteil kommt hinzu, daß die Solaranlage keine besondere Regelung erfordert, weil die am Dampfdrucktopf ohnehin erforderliche Regel- und Sicherheitseinrichtung zum Steuern des ganzen Systems heranzuziehen ist. Da das einem Dampfdrucktopf im allgemeinen zugeordnete Regelventil auf den Druck des im Topf enthaltenen Dampfes anspricht, wird bei der erfindungsgemäßen Einrichtung die Temperatur nicht nur im Dampfdrucktopf sondern auch in der Solaranlage über den Druck gesteuert.

Ein noch weiterer Vorteil der Erfindung besteht darin, daß der primäre Dampfkreislauf der Solaranlage über Schlauchkupplungen an den Dampfdrucktopf anzuschließen ist, die einen automatischen Verschluß beider Leitungsstücke besitzen. Zum erfindungsgemäßen Einbau in den primären Dampfkreislauf einer Solaranlage braucht ein genormter Dampfdrucktopf also nur mit je einer selbst schließenden Steckkupplung zum Anschluß der von der Solaranlage kommenden Dampfzuleitung zurückführenden Kondensat- bzw. Dampfleitung ausgestattet zu werden. Es bereitet keine Schwierigkeiten, solche Schlauchkupplungen an einem fertigen, genormten Dampfdrucktopf vorzusehen.

Nach erfolgter Sterilisation kann der Dampfdrucktopf vom solarbetriebenen Dampfkreislauf manuell abgekoppelt und an den Anwendungsort gebracht werden. Es ist nicht notwendig, den Topf zu öffnen und das sterilisierte Gut herauszunehmen, bevor es wirklich gebraucht wird. Die erfindungsgemäß vorgesehenen Schlauchkupplungen lassen es zu, einen frisch beladenen Dampfdrucktopf in den Kreislauf einzuschalten, ohne die Dampferzeugung im Solarteil zu unterbrechen. Hierdurch ist es möglich, die nur für etwa sieben bis acht Stunden pro Tag verfügbare Sonnenenergie optimal zu nutzen.

Um jedoch unzulässige Überhitzungen im Solarteil der Anlage zu vermeiden, kann der Dampf dann, wenn er nicht zum Sterilisieren benötigt wird, über einen Drei-Wege-Hahn zu einem luftgekühlten Kondensator bzw. Luft-Wärmetauscher geleitet und dort verflüssigt werden. Das Kondensat kann als destilliertes Wasser gesondert gesammelt oder in den Solarteil zurückgeleitet werden. Der Luft-Wärmetauscher kann alternativ auch als Wasser-Wärmetauscher ausgebildet werden. Dadurch läßt sich eine bessere Wärmeabfuhr und damit eine höhere Destillationsleistung erreichen und/oder die Wärme kann nutzbringend, z. B. zum Aufheizen von Brauchwasser, verwendet werden.

Ein weiterer wichtiger Vorteil des erfindungsgemäßen Sterilisators besteht darin, daß das Gerät wegen seines einfachen Aufbaus praktisch keine Möglichkeit zuläßt, durch Bedienungsfehler einen Unfall auszulösen. Sämtliche durch den Dampf erhitzten Teile können isoliert werden. Wegen der selbstschließenden Ventile in den Verbindungs- bzw. Schlauchkupplungen kann Dampf auch beim An- bzw. Entkuppeln nicht austreten. Wenn der parallel zu dem Dampfdrucktopf vorgesehene Luftkühler über ein temperaturgesteuertes Drei-Wege-Ventil in den solarbetriebenen Dampfkreislauf eingeschaltet wird, kann trotz Fehlens eines besonderen Regelventils in dem Solarteil der Anlage auch dann keine Überhitzung bzw. ein zu hoher Überdruck auftreten, wenn ein Dampfdrucktopf in den Kreislauf nicht eingeschaltet ist.

Anhand der schematischen Darstellung eines Ausführungsbeispiels eines an eine Solaranlagedirekt angeschlossenen medizinischen Sterilisators werden Einzelheiten der Erfindung erläutert.

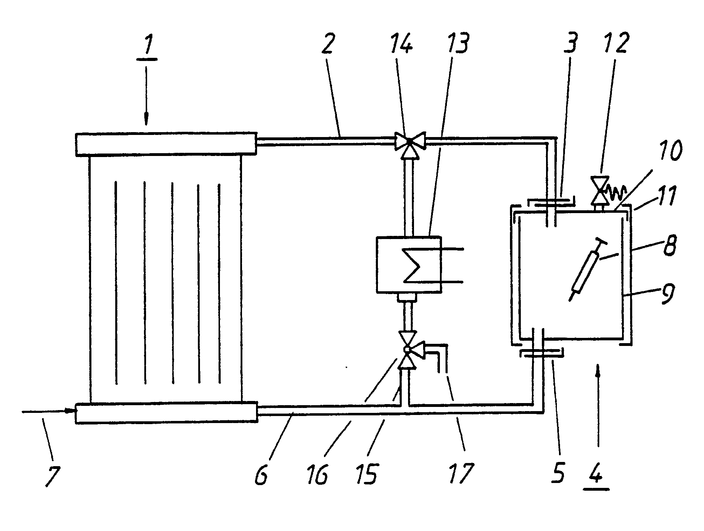

In dem in der Zeichnung dargestellten Ausführungsbeispiel wird in einer insgesamt mit 1 bezeichneten Solaranlage erzeugter Wasserdampf über eine Dampfzuleitung 2 und eine Schlauchkupplung 3 in einen insgesamt mit 4 bezeichneten Dampfdrucktopf geleitet und über eine weitere Schlauchkupplung 5 sowie eine Kondensat- bzw. Dampfableitung 6 zur Solaranlage 1 zurückgeführt. Die Solaranlage 1 soll bei der Anwendung zum direkten Einspeisen von Dampf in den Dampfdrucktopf 4 als Wärmeträger Flüssigkeit, Wasser, insbesondere Trinkwasser, enthalten. Zum Ergänzen von Trinkwasser in dem durch die Bauteile 1 bis 6 gebildeten Dampfkreislauf kann die Solaranlage 1 eine Wasserzuleitung 7 besitzen. Es kommen übliche Solaranlagen mit Hochtemperatur-Flachkollektor in Frage, die eine Dampftemperatur von 130° C und mehr erzeugen können. Die Anlagen sollen so ausgebildet bzw. ausgewählt werden, daß sie möglichst ohne Anpassung an den Sonnenstand während des größten Teils des Tages mit ausreichender Energieabgabe zu betreiben sind.

Als Dampfdrucktopf 4 kommt eine Vorrichtung mit einem die medizinischen Geräte 8 aufnehmenden Topf 9 mit Deckel 10 und Deckelverklammerung 11 in Frage. Der Dampfdrucktopf 4 soll außerdem mit einem Regel- und Sicherheitsventil 12 ausgestattet werden, das es erlaubt, über den Druck die Temperatur im Innern des Topfes zu steuern. Wenn die Solaranlage 1 über die Leitungen 2 und 6 an den Dampfdrucktopf 4 angeschlossen ist, wird auch der in ihr vorhandene Druck über das Regelventil 12 gesteuert.

Wenn es erwünscht ist, den jeweiligen Dampfdrucktopf 4 nach der Sterilisationszeit von z.B. 20 Minuten bei 130° C nicht zu öffnen sondern im geschlossenen Zustand an den Ort der Anwendung zu bringen, ist es wichtig, die Schlauchkupplungen 3 und 5 so auszubilden, daß sie beim Entkuppeln automatisch verschließen. Auch die an die Leitungen 2 und 6 angesetzten Kupplungsstücke sollen mit einem solchen selbst schließenden Ventil ausgestattet werden, damit der Druck in der Solaranlage 1 nicht beim Abkuppeln eines Dampfdrucktopfs 4 abfällt und/oder die Bedienungsperson durch ausströmenden Dampf verletzt werden kann.

Um eine Überhitzung oder einen zu großen Druck in der Solaranlage 1 beim Lösen der Schlauchkupplungen 3 und 5 zu vermeiden, kann parallel zu dem dem Dampfdrucktopf 4 zugeordneten Abschnitt des solaren Dampfkreislaufs ein Luftkühler 13 über ein Drei-Wege-Ventil 14 eingeschaltet werden. Das Ventil 14 kann vorzugsweise als temperaturgesteuertes Drei-Wege-Ventil zum alternativen Umschalten des solarbetriebenen Dampfkreislaufs vom Dampfdrucktopf 4 auf den Luftkühler 13 ausgebildet werden, derart, daß der Luftkühler 13 als Überhitzungsschutz dient.

Die Strecke des solarbetriebenen Dampfkreislaufs mit dem Luftkühler 13 kann aber auch als Luft-Wärmetauscher zum Erzeugen von destilliertem Wasser angewendet werden. Zu diesem Zweck kann in die Ableitung 15 des Lufterhitzers 13 ein Drei-Wege-Hahn 16 mit Ablauf 17 für das destillierte Wasser eingebaut werden. Dieses Hilfsteil der Anlage wird vorzugsweise dann aktiviert, wenn die jeweils zu absolvierende Sterilisationskampagne beendet ist und noch Sonnenscheindauer zur Energiegewinnung übrig geblieben ist. Bei Ausbildung des Luftkühlers als Wasser/Wasser-Wärmetauscher ergibt sich einersetis eine sehr gute Wärmeabfuhr und andererseits die Möglichkeit einer nutzbringenden Verwendung der Abwärme zum Aufheizen anderer Produkte, z. B. von Brauchwasser.

### Bezugszeichenliste

| | | |
|---|---|---|
| 1 | = | Solaranlage |
| 2 | = | Dampfzuleitung |
| 3 | = | Schlauchkupplung |
| 4 | = | Dampfdrucktopf |
| 5 | = | Schlauchkupplung |
| 6 | = | Dampfableitung |
| 7 | = | Wasserzuleitung |
| 8 | = | medizinisches Gut |
| 9 | = | Topf |
| 10 | = | Deckel |
| 11 | = | Deckelverklammerung |
| 12 | = | Regelventil |
| 13 | = | Luftkühler |
| 14 | = | Drei-Wege-Ventil |
| 15 | = | Ableitung |
| 16 | = | Drei-Wege-Hahn |
| 17 | = | Ablauf |

### Patentansprüche

1. Mit Solarenergie betriebener medizinischer Sterilisator mit einem als Dampfdrucktopf mit Druckregelventil ausgebildeten Sterilisationsbehälter, **dadurch gekennzeichnet,** daß der Dampfdrucktopf über einen automatischen Verschluß beider Leitungsstücke aufweisende Schlauchkupplungen in den primären Dampfkreislauf einer Solaranlage bei Steuerung des Gesamtsystems über das Druckregelventil des Dampfdrucktopfes unmittelbar eingeschaltet ist.

2. Sterilisator nach Anspruch 1, dadurch gekennzeichnet, daß der Dampfdrucktopf eine selbst schließende Steckkupplung zum Anschluß der von der Solaranlage kommenden Dampfzuleitung und der zur Solaranlage zurückführenden Kondensat- bzw. Dampfleitung besitzt.

3. Sterilisator nach Anspruch 1 oder 2, dadurch gekennzeichnet, daß parallel zu dem dem Dampfdrucktopf zugeordneten Abschnitt des solarbetriebenen Dampfkreislaufs ein Luftkühler oder Wasserkühler über ein Drei-Wege-Ventil einzuschalten ist.

4. Sterilisator nach Anspruch 3, dadurch gekennzeichnet, daß ein temperaturgesteuertes Drei-Wege-Ventil zum alternativen Umschalten des solarbetriebenen Dampfkreislaufs vom Dampfdrucktopf auf den Luftkühler oder Wasserkühler vorgesehen ist.

5. Sterilisator nach Anspruch 3 oder 4, dadurch gekennzeichnet, daß bei Verwendung von Wasser als Wärmeträger der dem Luftkühler zugeordnete Abschnitt des solarbetriebenen Dampfkreislaufs einen Drei-Wege-Hahn als Auslaß für destilliertes Waser enthält und daß die Solaranlage einen Wasserzulaufanschluß zum Ersetzen ablaufenden destillierten Wassers besitzt.

### Claims

1. With solar energy powered medical sterilizer with an sterilization autoclave constructed as pressure vessel with controlled pressure valve **characterized by** a pressure vessel which is connected directly to the primary steam circuit of a solar heat generator via a coupling mechanism with automatic with automatic shutter in both connections while the whole system is regulated by a pressure regulating valve of the autoclave.

2. Sterilizer according to claim 1, characterized by an automatically shutting and flexible hose compiling which serves to connect the steam pipe coming from the solar steam-generator and accordingly the condensate viz steam-pipe which leads back to the solar steam generator.

3. Sterilizer according to claim 1 and 2, characterized by a solar heated steam-circuit which could be paralleled by an air- or water-cooled condenser by activating a three-way valve.

4. Sterilizer according to claim 3, characterized by a temperature regulated three-way valve, which allows to alternatively switch the solar-driven steam circuit from the autoclave to the air- or water-cooled condenser.

5. Sterilizer according to claim 3 and 4, characterized by a three-way valve which can be used as outlet for distilled water in case that water as heat carrier is used and that the solar heat generator disposes of a water inlet for replacement of the produced distilled water.

## Revendications

1. Stérilisateur medical activé par energie solaire avec autoclave construit comme récipient préssurisé avec soupape régulateur, caractérisé en ce que, que l'autoclave dispose d'une connection automatique d'accouplement avec fermeture automatique, dont les deux gides connecté directement avec le circuit primaire à vapeur d'un générateur de chaleur solaire, et dont le système entier dispose d'une régulation de pression à l'aide d'une soupape régulateur intégrée dans l'autoclave.

2. Stérilisateur selon la revendication 1, caractérisé par un système de raccord express à fermetûre automatique qui sert à connecter l'autoclave et le conduite d'arrivé de vapeur venant de l'installation solaire et le conduite de condensé ou vapeur qui retourne à l'installation solaire.

3. Stérilisateur selon la revendication 1 ou 2, caracterisé par un condensateur réfrigeré à l'air ou à l'eau qui peut être mise en opération parallel au circuit solaire à vapeur, à l'aide d'un soupape à trois voies.

4. Stérilisateur selon la revendication 3, caractérisé par un soupape à trois voies, reglé par la temperature et qui sert à connecter ou disconnecter alternativement le circuit à vapeur solaire de l'autoclave ou bien le condensateur à l'air ou à l'eau.

5. Stérilisateur selon la revendication 3 ou 4, caracterisé par un soupape à trois voies, dans la section du circuit à vapeur qui mène vers le condensateur qui sert comme arrivé pour l'eau destillée et par un accouplement qui sert à ammener de l'eau à l'installation solaire pour remplacer l'eau destillée soustraite.